# EUROPEAN PATENT APPLICATION

(11) **EP 2 156 835 A1**
(43) Date of publication of application: **24.02.2010**
(21) Application number: 08290787.4
(22) Date of filing: 19.08.2008
(51) Int. Cl.: A61K 31/4523, A61P 13/00

(54) **New therapeutic use of drotaverine**

(71) Applicant: Sanofi-Aventis, 75013 Paris (FR)
(72) Inventor: LLuel, Philippe, 31520 Ramnoville Saint Agne (FR); Palea, Stefano, 31400 Toulouse (FR); Sofeir, Maurice, Paris (FR)
(74) Representative: Weber, Mathieu

(57) **Abstract**

The subject of the present invention is a new use of drotaverine, in the free state or in the form of a salt, for the preparation of a medicament intended for the treatment or amelioration of benign prostatic hyperplasia, urinary disorders, disorders related to bladder dysfunction, lower urinary tract symptoms associated or not associated with benign prostatic hyperplasia, urinary incontinence, bladder outlet obstruction associated or not associated with benign prostatic hyperplasia, interstitial cystitis and overactive bladder.

## Description

The subject of the present invention is a new therapeutic use of drotaverine.

Drotaverine (or 1-[(3,4-diethoxyphenyl)methylene]-6,7-diethoxy-1,2,3,4-tetrahydro-isoquinoline) is a known antispasmodic drug and is commercialized under the trademark No-spa® mainly in some countries of Eastern and Central Europe, and Sub Saharan Africa.

We have now found, quite surprisingly and unexpectedly, that drotaverine is efficient and advantageous in ameliorating and/or treating benign prostatic hyperplasia, urinary disorders, disorders related to bladder dysfunction, lower urinary tract symptoms associated or not associated with benign prostatic hyperplasia, urinary incontinence, bladder outlet obstruction associated or not associated with benign prostatic hyperplasia, interstitial cystitis and overactive bladder.

Therefore, an object of the present invention is the use of drotaverine, in the free state or in the form of a salt, for the preparation of a medicament intended for the treatment or amelioration of benign prostatic hyperplasia, urinary disorders, disorders related to bladder dysfunction, lower urinary tract symptoms associated or not associated with benign prostatic hyperplasia, urinary incontinence, bladder outlet obstruction associated or not associated with benign prostatic hyperplasia, interstitial cystitis and overactive bladder.

The term urinary incontinence includes mixed-incontinence, urge-incontinence, stress-incontinence, functional incontinence and overflow incontinence.

According to the invention, drotaverine is present in the free state or in the form of a salt.

These salts include for example salts with mineral acids such as hydrochloric acid, hydrobromic acid, sulfuric acid, nitric acid, phosphoric acid; salts with organic acids such as methanesulfonic acid, benzenesulfonic acid, p-toluenesulfonic acid, acetic acid, propionic acid, tartaric acid, fumaric acid, maleic acid, malic acid, oxalic acid, succinic acid, citric acid, benzoic acid, mandelic acid, cinnamic acid, lactic acid, glycolic acid, glucuronic acid, ascorbic acid, nicotinic acid, and salicylic acid; or salts with acidic amino acids such as aspartic, and glutamic acid. The preferred salt of drotaverine is drotaverine hydrochloride.

Therefore, another object of the present invention is the use of drotaverine hydrochloride for the preparation of a medicament intended for the treatment or amelioration of benign prostatic hyperplasia, urinary disorders, disorders related to bladder dysfunction, lower urinary tract symptoms associated or not associated with benign prostatic hyperplasia, urinary incontinence, bladder outlet obstruction associated or not associated with benign prostatic hyperplasia, interstitial cystitis and overactive bladder.

A further object of the present invention is a use as describe above for the treatment or amelioration of overactive bladder.

A further object of the present invention is a use as describe above for the treatment or amelioration of urinary incontinence.

A further object of the present invention is a use of a pharmaceutical composition comprising drotaverine, in the free state or in the form of a salt, for the preparation of a medicament intended for the treatment or amelioration of benign prostatic hyperplasia, urinary disorders, disorders related to bladder dysfunction, lower urinary tract symptoms associated or not associated with benign prostatic hyperplasia, urinary incontinence, bladder outlet obstruction associated or not associated with benign prostatic hyperplasia, interstitial cystitis and overactive bladder.

These pharmaceutical compositions are preferably made so as to be administered by the oral or parenteral route.

In the pharmaceutical compositions of the present invention for oral, sublingual, subcutaneous, intramuscular, intravenous, intradermal, local or rectal administration, the active ingredient may be administered in unit forms for administration, mixed with conventional pharmaceutical carriers, to animals and to human beings. For example, the pharmaceutical composition may be formulated, for example, in the form of pharmaceutical compositions for oral administration such as granules, fine granules, powders, hard capsules, soft capsules, syrups, emulsions, suspensions, solutions and the like, or in the form for sublingual a buccal administration, or in the form of pharmaceutical compositions for parenteral administrations such as injections for intravenous, intramuscular, or subcutaneous administration, drip infusions, transdermal preparations, transmucosal preparations, nasal drops, inhalants, suppositories and the like. Injections or drip infusions may be prepared as powdery preparations such as in the form of lyophilized preparations, and may be used by dissolving just before use in an appropriate aqueous medium such as physiological saline. Sustained-release preparations such as those coated with a polymer may be directly administered intracerebrally.

Types of pharmaceutical additives used for the manufacture of the pharmaceutical composition, content ratios of the pharmaceutical additives relative to drotaverine or its salts, and methods for preparing the pharmaceutical composition may be appropriately chosen by those skilled in the art. Inorganic or organic substances or solid or liquid substances may be used as pharmaceutical additives. Generally, the pharmaceutical additives may be incorporated in a ratio ranging from 1% by weight to 90% by weight based on the weight of drotaverine or its salts.

Examples of excipients used for the preparation of solid pharmaceutical compositions include, for example, lactose, sucrose, starch, talc, cellulose, dextrin, kaolin, calcium carbonate and the like. When a solid composition in the form of tablets is prepared, the active ingredients are mixed with the excipients. The tablets can be coated with sucrose or other appropriate materials or alternatively they can be treated such that they have a prolonged or delayed activity and that they have a prolonged or delayed activity and that they continuously liberate a predetermined quantity of active ingredient.

A preparation in the form of gelatin capsules is obtained by mixing the active ingredient with a diluent and by pouring the mixture obtained into soft or hard gelatin capsules. For the preparation of liquid compositions for oral administration, a conventional inert diluent such as water or a vegetable oil may be used. The liquid composition may contain, in addition to the inert diluent, auxiliaries such as moistening agents, suspension aids, sweeteners, aromatics, colorants, and preservatives. The liquid composition may be filled in capsules made of an absorbable material such as gelatin. Examples of solvents or suspension mediums used for the preparation of compositions for parenteral administration, e.g. injections, suppositories, include water, propylene glycol, polyethylene glycol, benzyl alcohol, ethyl oleate, lecithin and the like. Examples of base materials used for suppositories include, for example, cacao butter, emulsified cacao butter, lauric lipid, witepsol.

The dose and frequency of administration of the medicament of the present invention are not particularly limited, and they may be appropriately chosen depending on conditions such as the body weight or age of a patient, severity and the like. Generally, a daily dose for oral administration may be administered once a day or several times a day as divided portions, or once in several days.

In the text, which follows, the quantity of drotaverine is expressed as drotaverine equivalents in non-salified, free form.

When the composition of the invention is administered in man by the parenteral or oral route, the daily dose of drotaverine may vary between 15 to 250 mg. Preferably the daily dose of drotaverine may vary between 120 to 240 mg. More preferably, the dose of drotaverine hydrochloride can be 40 mg once to six times per day, or 80 mg, once to three times per day.

It will be noted that according to the invention, drotaverine or its salts can be administered by the oral route, or by the intramuscular route or by the intravenous route.

A further object of the present invention is a method for treating/ameliorating the pathologies indicated above, which comprises the administration to a patient of an effective amount of drotaverine or its salts according to the invention.

A further object of the present invention is a method for treating/ameliorating the pathologies indicated above, which comprises the administration to a patient of an effective amount of a composition comprising drotaverine or its salts according to the invention.. The following example is an illustration of the present invention.

### Example 1: Effect of drotaverine on the induced plateau of contraction in human isolated detrusor muscle

We studied the effect of drotaverine on the induced plateau of contraction in human isolated detrusor muscle.

The detrusor muscle was separated from mucosa and adventitia and cut into small strips which were mounted, under 1 g initial tension, in 5 mL glass organ baths containing oxygenated Krebs-Henseleit solution (composition in mM: NaCl 114, KCl 4.7, CaCl2 2.5, MgS04 1.2, KH2PO4 1.2, NaHCO3 25, glucose 11.7) kept at 37°C. Tissues were allowed to equilibrate for at least 60 minutes during which time the Krebs solution was replaced every 15 min. After the stabilization period, a reference contraction to 80 mM KCl was performed on each strip. Tissues having a contraction less than 1 g were discarded. After wash-outs and a further 45 min of re-equilibration, during which the resting tension was adjusted periodically, each strip was primed with 0.1 µM forskolin, an adenylyl cyclise activator, for 10 min then recontracted with 1 µM U46619 for at least 20 min, until a stable plateau of contraction was obtained. Strips having a plateau of contraction less than 0.2 g were discarded. After obtaining a stable plateau of contraction, concentration-responses curves (CRC) to drotaverine and rolipram (0.03 - 100 µM) or the common vehicle (DMSO from 0.003 to 1 % in the organ bath) were performed. At the end of the CRC, the maximal relaxant effect for each strip was determined following application of 10 µM forskolin into the organ baths. Only one CRC to each substance or solvent was tested in a single strip. CRCs were constructed using 8 bladder strips from 4 different patients. Results were expressed as percent relaxation of the plateau of contraction induced by 1 µM U46619, with respect to the maximal effect obtained with 10 µM forskolin taken as 100% relaxation.

All CRCs were fitted by non linear regressions using the software GraphPad Prism® version 4.0 to obtain the following parameters: Emax: Maximal relaxation induced by the tested substance; EC50: the concentration which induces 50% of the maximum effect, expressed as pEC50 (-log EC50). Comparisons of the CRCs were performed with two-way analysis of variance (ANOVA) statistical analysis test and Bonferroni post-test. All differences will be considered statistically significant when the null hypothesis can be rejected at a risk α of less than 0.05.

All the strips tested produced a contraction to 80 mM KCl greater than 1 g. The magnitude of contractions (grams of tension) induced by 80 mM KCI were the following: 7.42 ± 1.29, 7.71 ± 1.97 and 6.35 ± 0.95 for strips used to test drotaverine, rolipram and solvent, respectively. These values were compared by one-way ANOVA and the Kruskall-Wallis test and no statistically significant difference was observed. The thromboxane receptor agonist, U46619 at the concentration of 1 µM, induced a sustained plateau of contraction with a magnitude of 1.43 ± 0.34, 2.07 ± 0.79 and 1.16 ± 0.29, for strips used to test drotaverine, rolipram and solvent, respectively. These values were compared by one-way ANOVA and the Kruskall-Wallis test and no statistically significant difference was observed. However, in the patient 6, all the strips tested exhibited abnormally low contractile responses to U46619 (between 0.2 and 0.4 g), so we decided to exclude all the results obtained from this patient. When the strips were incubated with the DMSO from 0.0003 to 1 % (v/v) in the organ bath, this solvent induced a slight relaxation of U46619-contracted human bladder only at the highest volume added.

The maximal relaxant effect (observed at 1% DMSO) was 36.08 ± 3.73 %. Drotaverine, from 0.03 to 10 µM induced a slight relaxation of U46619-contracted detrusor strips. However from 30 to 100 µM, drotaverine induced a significant concentration-dependent relaxation of U46619-induced detrusor muscle contraction. Fitting the drotaverine CRC (n=10) indicated a mean pEC50 of 4.49 ± 0.05 and a maximal relaxant effect (Emax) of 80.79 ± 3.07 % at the concentration of 100 µM. Rolipram, from 0.03 to 100 µM, induced a significant concentration-dependent relaxation of U46619-induced human detrusor contraction. Fitting the rolipram CRC (n=11) indicated a mean pEC50 of 6.82 ± 0.12 and a maximal relaxant effect (Emax) of 89.53 ± 2.68 % at the concentration of 100 µM.

These results show that drotaverine induces a significant concentration-dependant relaxation of the human detrusor muscle contraction.

Because spontaneaous non-voiding contractions observed during cystometry sessions of men with overactive bladder have been linked to spontaneous phasic contractions of human bladder strips but also animals having an experimental overactive bladder (Sibley G.N., 1984; Mills I.W. et al., 2000), these results support the new use according to the present invention.

## Claims

1. Use of drotaverine, in the free state or in the form of a salt, for the preparation of a medicament intended for the treatment or amelioration of benign prostatic hyperplasia, urinary disorders, disorders related to bladder dysfunction, lower urinary tract symptoms associated or not associated with benign prostatic hyperplasia, urinary incontinence, bladder outlet obstruction associated or not associated with benign prostatic hyperplasia, interstitial cystitis and overactive bladder.

2. Use according to claim 1, of drotaverine hydrochloride.

3. Use according to claim 1 or 2, in which the treatment involves the administration by the parenteral and/or oral route of 15 to 250 mg of drotaverine per day.

4. Use according to any one of the preceding claims for the preparation of a medicament intended for the treatment or amelioration of overactive bladder.

5. Use according to any one of claims 1 to 3 for the preparation of a medicament intended for the treatment or amelioration of urinary incontinence.
